# EUROPEAN PATENT APPLICATION

(11) **EP 4 349 441 A1**
(43) Date of publication of application: **10.04.2024**
(21) Application number: 22819501.2
(22) Date of filing: 07.06.2022
(51) Int. Cl.: B01D 3/14, C07D 317/36

(54) **CONTINUOUS RECTIFICATION APPARATUS FOR PRODUCING ELECTRONIC-GRADE PROPYLENE CARBONATE**

(30) Priority: 07.06.2021 CN 202110631688
(71) Applicant: Shandong Lixing Chemical Co., Ltd., Linyi, Shandong 276100 (CN)
(72) Inventor: ZHANG, Junkai, Tancheng County Linyi, Shandong 276100 (CN); ZHANG, Guangzhi, Tancheng County Linyi, Shandong 276100 (CN); SU, Qiangde, Tancheng County Linyi, Shandong 276100 (CN)
(74) Representative: 2SPL Patentanwälte PartG mbB
(86) International application number: PCT/CN2022/097295
(87) International publication number: WO 2022/257903

(57) **Abstract**

The present invention relates to the technical field of propylene carbonate production. Specifically disclosed is a continuous rectification apparatus for producing electronic-grade propylene carbonate, comprising a light component removal tower and a heavy component removal tower. One side of the light component removal tower is connected to a first feeding pipe. The top of the light component removal tower is connected to a first condensing box by means of a pipeline. One side of the first condensing box is connected to a first reflux tank by means of a pipeline. One side of the first reflux tank is connected to a first conveying pump by means of a pipeline. One end of the first conveying pump is connected to a first reflux pipe. The bottom of the light component removal tower is connected to a feeding pump by means of a pipeline. One end of the feeding pump is connected to a second feeding pipe. One end of the second feeding pipe is connected to one side of the heavy component removal tower. The top of the heavy component removal tower is connected to a second condensing box by means of a pipeline. One side of the second condensing box is connected to one side of a second reflux tank by means of a pipeline. An electronic-grade propylene carbonate product is obtained through the rectification and separation of a propylene carbonate product by means of the light component removal tower and the heavy component removal tower, and continuous rectification conserves energy.

## Description

### TECHNICAL FIELD

The present disclosure relates to the technical field of propylene carbonate production, in particular to a continuous rectification apparatus for producing electronic-grade propylene carbonate.

### BACKGROUND

Propylene carbonate is a colorless and odorless flammable liquid. The propylene carbonate is miscible with ether, acetone, benzene, chloroform, vinyl acetate and the like and soluble in water and carbon tetrachloride, and has strong absorption capacity for carbon dioxide and stable properties. The existing propylene carbonate needs to be processed in a rectification column. The existing rectification column is used for a single distillation, and crude liquid is not purified again, which results in poor distillation effect and affects the quality of products.

### SUMMARY

The present disclosure aims to provide a continuous rectification apparatus for producing electronic-grade propylene carbonate, so as to solve the problems proposed in the background.

In order to achieve the above purpose, the present disclosure provides the following technical solution. A continuous rectification apparatus for producing electronic-grade propylene carbonate includes a light component removal column and a heavy component removal column. One side of the light component removal column is connected to a first feeding pipe. A top of the light component removal column is connected to a first condensing box via a pipeline. One side of the first condensing box is connected to a first reflux tank via a pipeline. One side of the first reflux tank is connected to a first conveying pump via a pipeline. One end of the first conveying pump is connected to a first reflux pipe. A bottom of the light component removal column is connected to a feeding pump via a pipeline. One end of the feeding pump is connected to a second feeding pipe. One end of the second feeding pipe is connected to one side of the heavy component removal column. A top of the heavy component removal column is connected to a second condensing box via a pipeline. One side of the second condensing box is connected to one side of a second reflux tank via a pipeline. Another side of the second reflux tank is connected to one end of a second conveying pump via a pipeline. Another end of the second conveying pump is connected to a second reflux pipe. One end of the second reflux pipe is connected to another side of the heavy component removal column. The second reflux pipe is connected to a second extraction pipe.

Preferably, the first reflux pipe is connected with a first extraction pipe. One end of the first extraction pipe is connected with a first collecting tank.

Preferably, one end of the second extraction pipe is connected with a second collecting tank. The second extraction pipe and the first extraction pipe are provided with valves.

Compared with the prior art, the present disclosure has beneficial effects that an electronic-grade propylene carbonate product is obtained by means of the rectification and separation of a propylene carbonate product via the light component removal column and the heavy component removal column, and continuous rectification conserves energy. The first reflux tank and the second reflux tank are configured to reflux the rectified product to the light component removal column and the heavy component removal column for processing again, so that the accuracy of the product is improved.

### BRIEF DESCRIPTION OF THE DRAWINGS

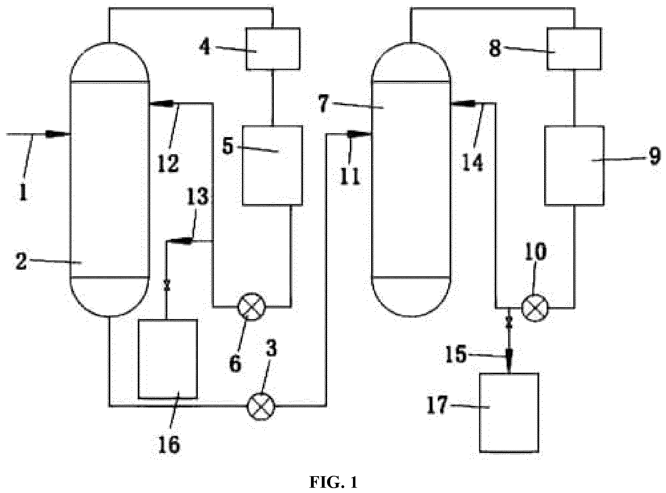

FIG. 1 is an overall structural schematic diagram of the present disclosure.

Reference signs: 1, first feeding pipe; 2, light component removal column; 3, feeding pump; 4, first condensing box; 5, first reflux tank; 6, first conveying pump; 7, heavy component removal column; 8, second condensing box; 9, second reflux tank; 10, second conveying pump; 11, second feeding pipe; 12, first reflux pipe; 13, first extraction pipe; 14, second reflux pipe; 15, second extraction pipe; 16, first collecting tank; and 17, second collecting tank.

### DETAILED DESCRIPTION OF THE EMBODIMENTS

The following clearly and completely describes the technical solutions in the embodiments of the present disclosure with reference to the accompanying figures in the embodiments of the present disclosure. Apparently, the described embodiments are merely a part rather than all of the embodiments of the present disclosure. Based on the embodiment in the present disclosure, all other embodiments obtained by those of ordinary skill in the art under the premise of without contributing creative labor belong to the scope of the present disclosure.

In the description of the present disclosure, it needs to be noted that the indicative direction or position relations of the terms such as "vertical", "upper", "lower" and "horizontal" are direction or position relations illustrated based on the accompanying figures, just for facilitating the description of the present disclosure and simplifying the description, rather than indicating or hinting that the indicated apparatus or element must be in a specific direction and constructed and operated in the specific direction, thus the terms cannot be understood as the restriction of the present disclosure.

In the description of the present disclosure, it needs to be noted that the terms such as "arrange", "install", "link" and "connect" should be generally understood unless otherwise specified and limited, for example, the components can be fixedly connected, and also can be detachably connected or integrally connected; the components can be mechanically connected, and also can be electrically connected; the components can be directly connected and also can be indirectly connected through an intermediate, and two components can be communicated to one another internally. For those of ordinary skill in the art, the specific meanings of the terms in the present disclosure can be understood according to specific conditions.

Referring to FIG. 1, provided is a continuous rectification apparatus for producing electronic-grade propylene carbonate, including a light component removal column 2 and a heavy component removal column 7. One side of the light component removal column 2 is connected to a first feeding pipe 1 for facilitating a propylene carbonate product to enter the light component removal column 2, so as to perform light component removal processing. The top of the light component removal column 2 is connected to a first condensing box 4 via a pipeline for cooling the refluxed product. One side of the first condensing box 4 is connected to a first reflux tank 5 via the pipeline. The first reflux tank 5 collects the condensed product. One side of the first reflux tank 5 is connected to a first conveying pump 6 via the pipeline. One end of the first conveying pump 6 is connected to a first reflux pipe 12. The first conveying pump 6 conveys the refluxed product in the first reflux tank 5 into the light component removal column 2 through the first reflux pipe 12 for light component removal processing.

The bottom of the light component removal column 2 is connected to a feeding pump 3 via the pipeline. One end of the feeding pump 3 is connected to a second feeding pipe 11. One end of the second feeding pipe 11 is connected to one side of the heavy component removal column 7. The feeding pump 3 conveys the product after light component removal into the heavy component removal column 7 for heavy component removal processing. The top of the heavy component removal column 7 is connected to a second condensing box 8 via the pipeline for cooling the refluxed product. One side of the second condensing box 8 is connected to one side of a second reflux tank 9 via the pipeline. The other side of the second reflux tank 9 is connected to one end of a second conveying pump 10 via the pipeline. The other end of the second conveying pump 10 is connected to a second reflux pipe 14. One end of the second reflux pipe 14 is connected to the other side of the heavy component removal column 7. The second conveying pump 10 conveys the refluxed product in the second reflux tank 9 into the heavy component removal column 7 through the second reflux pipe 14 for heavy component removal processing. The second reflux pipe 14 is connected to a second extraction pipe 15 for facilitating outputting the product separated by continuous rectification.

The first reflux pipe 12 is connected with a first extraction pipe 13. One end of the first extraction pipe 13 is connected with a first collecting tank 16. The first collecting tank 16 collects the product flowing out of the first extraction pipe 13.

One end of the second extraction pipe 15 is connected with a second collecting tank 17. The second extraction pipe 15 and the first extraction pipe 13 are provided with valves. The second collecting tank 17 collects the product flowing out of the second extraction pipe 15.

The working principle is as follows. When in use, the propylene carbonate product is conveyed into the light component removal column 2 via the first feeding pipe 1 for the light component removal, the product after light component removal enters the pipeline. After the rest product is all refluxed into the first condensing box 4 for condensation, the refluxed product in the first condensing box 4 enters the first reflux tank 5, is conveyed by the first conveying pump 6, enters the first reflux pipe 12, and then is refluxed into the light component removal column 2. The product after light component removal is output into the first collecting tank 16 for collection via the first extraction pipe 13. The product after light component removal is conveyed into the second feeding pipe 11, and then enters the light component removal column 7 by means of the operation of the feeding pump 3 for heavy component removal. Materials after heavy component removal are refluxed into the pipeline. By means of the operation of the second conveying pump 10, the refluxed materials firstly enter the second condensing box 8 for condensation treatment, then enter the second reflux tank 9 to be refluxed into the second reflux pipe 14, and then enter the heavy component removal column 7 for heavy component removal processing again. The product separated by continuous distillation is conveyed into the second collecting tank 17 for collection via the second extraction pipe 15.

Although the embodiments of the present disclosure have already been illustrated and described, various changes, modifications, replacements and transformations can be made by those of ordinary skill in the art under the condition of without departing from the principle and the spirit of the present disclosure, and thus the scope of the present disclosure should be restricted by claims and equivalents thereof.

## Claims

1. A continuous rectification apparatus for producing electronic-grade propylene carbonate, comprising a light component removal column (2) and a heavy component removal column (7), wherein one side of the light component removal column (2) is connected to a first feeding pipe (1), a top of the light component removal column (2) is connected to a first condensing box (4) via a pipeline, one side of the first condensing box (4) is connected to a first reflux tank (5) via a pipeline, one side of the first reflux tank (5) is connected to a first conveying pump (6) via a pipeline, one end of the first conveying pump (6) is connected to a first reflux pipe (12), a bottom of the light component removal column (2) is connected to a feeding pump (3) via a pipeline, one end of the feeding pump (3) is connected to a second feeding pipe (11), one end of the second feeding pipe (11) is connected to one side of the heavy component removal column (7), a top of the heavy component removal column (7) is connected to a second condensing box (8) via a pipeline, one side of the second condensing box (8) is connected to one side of a second reflux tank (9) via a pipeline, another side of the second reflux tank (9) is connected to one end of a second conveying pump (10) via a pipeline, another end of the second conveying pump (10) is connected to a second reflux pipe (14), one end of the second reflux pipe (14) is connected to the other side of the heavy component removal column (7), and the second reflux pipe (14) is connected to a second extraction pipe (15).

2. The continuous rectification apparatus for producing electronic-grade propylene carbonate according to claim 1, wherein the first reflux pipe (12) is connected to a first extraction pipe (13), and one end of the first extraction pipe (13) is connected to a first collecting tank (16).

3. The continuous rectification apparatus for producing electronic-grade propylene carbonate according to claim 1, wherein one end of the second extraction pipe (15) is connected to a second collecting tank (17), and both the second extraction pipe (15) and the first extraction pipe (13) are provided with valves.
